# EUROPEAN PATENT APPLICATION

(11) **EP 4 667 484 A1**
(43) Date of publication of application: **24.12.2025**
(21) Application number: 23922096.5
(22) Date of filing: 13.03.2023
(51) Int. Cl.: C07K 14/00, C07K 19/00, C12N 15/09, C12N 15/117, A61K 38/16, A61P 37/00, A61P 3/10

(54) **HOMODIMERIC PROTEIN COMBINING HUMAN IL-1R1 WITH HUMAN IL-1RACP POLYPEPTIDE FRAGMENTS AND USE**

(30) Priority: 13.02.2023 CN 202310105416
(71) Applicant: QIZHEN BIOPHARMA CO., LTD. (HANGZHOU), Hangzhou, Zhejiang 311231 (CN)
(72) Inventor: ZHOU, Qing, Hangzhou, Zhejiang 310058 (CN); WANG, Yusha, Hangzhou, Zhejiang 310058 (CN); YU, Xiaomin, Hangzhou, Zhejiang 310058 (CN)
(74) Representative: Gramm, Lins & Partner Patent- und Rechtsanwälte PartGmbB
(86) International application number: PCT/CN2023/081176
(87) International publication number: WO 2024/168965

(57) **Abstract**

A homodimeric protein combining human IL-1R1 with human IL-1RAcP polypeptide fragments and the use. A human IL-1R1 gene has a mutation at a single nucleotide site on the basis of a wild type, i.e., a nucleotide of a coding K131 site is mutated from AAA to GAA, so that a K131E mutation occurs in a translationally expressed IL-1R1 protein. A homodimeric protein, also known as an IL-1 trap protein, specifically binds to IL-1α and/or IL-1β and comprises two homologous polypeptide sequences, wherein each polypeptide sequence comprises three segments of amino acid sequences. The first segment of amino acid sequence comprises amino acids at positions 21 to 359 of human L-1RAP; the second segment of amino acid sequence comprises amino acids at positions 18 to 332 of human IL-1R1, wherein the amino acid at position 131 is mutated from Lys to Glu or Asp, i.e., a K131E or K131D mutation; and the third segment of amino acid sequence comprises a sequence of human immunoglobulin gamma-1 Fc. The IL-1 trap protein can not only specifically block the function of inflammatory activator IL-1α or IL-1β, but can also retain the function of inflammatory antagonist IL-1Ra, thereby accelerating the speed of inhibiting inflammation.

## Description

### TECHNICAL FIELD

The present disclosure relates to the field of biomedicine and the fields of genetic engineering and protein engineering; and more specifically, the present disclosure relates to a homodimeric protein combining a human IL-1R1 polypeptide fragment containing a special mutation with a human IL-1RAcP polypeptide fragment, and use thereof, where the use includes preparation and application of polypeptide medicaments; the application includes treatment or prevention of diseases related to overexpression and activation of IL-1α or IL-1β; and the homodimeric protein can selectively bind to inflammatory factors IL-1α and IL-1β without binding to an inflammatory inhibitory factor IL-Ra, which significantly improves the efficiency of inhibiting IL-1 signaling pathway.

### BACKGROUND

### Interleukin-1 Family and Receptors Thereof

Interleukin-1 (IL-1) affects nearly all cells and organs, and is a major pathogenic mediator in autoinflammatory, autoimmune, infectious, and degenerative diseases.

The IL-1 family includes 11 members, including 7 ligands with agonist activity (IL-1α, IL-1β, IL-18, IL-33, IL-36α, IL-36β, and IL-36γ), three receptor antagonists (IL-1Ra, IL-36Ra, and IL-38), and an anti-inflammatory cytokine (IL-37). The IL-1 receptor (IL-1R) family includes 10 structurally related members (IL-1R1, IL-1R2, IL-1RAcP, IL-18Rα, IL-18Rβ, ST2, IL-1Rrp2, TIR8, TIGIRR-2, and TIGIRR-1), and a distantly related soluble protein IL-18BP acting as an inhibitor of the cytokine IL-18.

### Activation of IL-1R1 Pathway

In the IL-1 pathway, soluble cytokines IL-1α and IL-1β interact with the extracellular domain of IL-1R1, to trigger recruitment of the co-receptor IL-1RAcP, thereby forming a functional receptor complex for initiating the IL-1R1 signaling cascade. The heterotrimeric IL-1/IL-1R1/IL-1RAcP complex causes dimerization of the TIR domains of IL-1R1 and IL-1RAcP proteins, to provide an anchor point for recruitment of myeloid differentiation primary response protein 88 (Myd88). This protein-protein interaction triggers recruitment of other signaling molecules, for example IL-1R-associated kinase (IRAK) and TNF receptor-associated factor 6 (TRAF6), to the protein complex. Subsequently, multiple intracellular phosphorylation and ubiquitination processes ultimately cause activation of mitogen-activated protein kinase (MAPK) p38, c-Jun N-terminal kinase (JNK), and nuclear factor kappa B (NF-κB). These changes cause upregulation of mRNA transcription of inflammation-related genes.

In addition to the agonists IL-1α and IL-1β, IL-1R1 also binds to endogenous antagonist IL-1Ra. IL-1Ra cannot trigger binding of IL-1R1 to IL-1RAcP, to competitively block IL-1 signaling by binding to IL-1R1. In addition, when IL-1β binds to IL-1R2, no signaling occurs because IL-1R2 lacks a cytoplasmic TIR domain. IL-1 signaling can also be inhibited by the receptors sIL-1R1 and sIL-1R2 in soluble forms, which lack transmembrane and intracellular regions in native forms and cannot mediate signaling.

### IL-1α and Related Diseases Thereof

IL-1α is a classic damage-associated molecular pattern (DAMP) molecule and acts as an "alarm" and a local inflammatory mediator. A precursor of IL-1α also has biological activity. The constitutive precursor of IL-1α exists in all healthy tissues of mesenchymal origin, for example keratinocytes in the skin, type 2 epithelial cells in the lung, epithelial cells in the whole gastrointestinal tract, endothelial cells in blood vessels, and astrocytes in the brain. After cell necrosis caused due to factors such as trauma, ischemia, or viral infections, the IL-1α precursor is immediately released into the extracellular space and then binds to IL-1R1 in adjacent living cells, to stimulate production of chemokines and cause infiltration of neutrophils and monocytes, thereby inducing tissue inflammation.

In human diseases, IL-1α disrupts barrier epithelial cells (i.e., skin diseases, airway diseases, and colitis), induces endothelial damage, or promotes thrombosis (i.e., Behçet's syndrome and vasculitis), causing inflammatory responses.

Human diseases involving IL-1α can be classified into the following categories:
(1) Skin diseases, for example, purulent edema, other neutrophilic dermatoses, Hidradenitis Suppurativa, and Systemic Sclerosis;
(2) Lung and respiratory diseases, for example, Chronic Obstructive Pulmonary Disease (COPD), Pulmonary Fibrosis, and Cystic Fibrosis;
(3) Gastrointestinal diseases, for example, colitis;
(4) Muscle tissue diseases, for example, Dermatomyositis and Polymyositis;
(5) Cardiovascular diseases, for example, Myocarditis, Pericarditis, and Behçet's Syndrome; and
(6) Cancer. Neutralizing monoclonal antibodies targeting IL-1α have been used in clinical trials for the treatment of advanced NSCLC (Non-Small Cell Lung Cancer) and advanced metastatic colorectal cancer.

### IL-1β and Related Diseases Thereof

IL-1β is a typical pro-inflammatory factor capable of activating the IL-1R1 signaling pathway and initiating expression of inflammation-related genes, for example, pro-inflammatory cytokines and chemokines. Different from IL-1α, IL-1β is not synthesized in cells under homeostatic conditions and is generated only by some cells (for example, blood monocytes, tissue macrophages, and dendritic cells) upon stimulation. Stimuli can be microbial products, and cytokines such as tumor necrosis factor (TNF), IL-18, IL-1α, or IL-1β can also induce transcription of IL-1β. The precursor of IL-1β is biologically inactive. To process the precursor of IL-1 β, activation of Caspase 1 needs to be induced by activation of inflammasomes, and the activated Caspase 1 processes the precursor of IL-1β into active IL-1β.

Human diseases involving IL-1β can be classified into the following categories:
(1) Classical autoinflammatory diseases; classical autoinflammatory diseases are rare genetic disorders, characterized by enhanced activation of inflammasomes, increased secretion of IL-1β, and exacerbated inflammations typically caused by gene mutations, for example, Familial Mediterranean Fever (FMF) and Cryopyrin-Associated Periodic Syndromes (CAPS);
(2) Systemic inflammatory diseases, for example, Systemic Juvenile Idiopathic Arthritis (SJIA), Periodic Fever, Aphthous Stomatitis, Pharyngitis, Adenitis and Syndrome (PFAPA), and Schnitzler Syndrome;
(3) Joint, bone and muscle diseases, for example, Rheumatoid Arthritis, Ankylosing Spondylitis, and Recurrent Multifocal Osteomyelitis;
(4) Common inflammatory diseases, for example, Gout/Pseudogout and type 2 Diabetes Mellitus; and
(5) Cancer. IL-1β induces tumor angiogenesis and metastatic spread of tumors, for example, promoting bone metastasis of breast cancer cells.

### IL-1Ra and Related Diseases Thereof

IL-1Ra, as an endogenous antagonist of IL-1R1, blocks IL-1 signaling by binding to IL-1R1 in competition with IL-1α and IL-1β. Deficiency of the Interleukin-1-Receptor Antagonist (DIRA) is a monogenic genetic disorder, and variations in IL-1RN cause the deficiency of IL-1Ra. Patients have symptoms of sterile multifocal osteomyelitis, periostitis, and pustulosis.

Recombinant IL-1Ra protein used as an anti-inflammatory drug has been commercialized as Anakinra, sold under a trade name "Kineret" (see US5075222). Anakinra has been approved for the treatment of Rheumatoid Arthritis, Neonatal-Onset Multisystem Inflammatory Disease (NOMID), and DIRA. A disadvantage of Anakinra is that it is delivered as an injection concentrate at a dose of 100 mg with a short half-life, and therefore, needs to be injected daily.

### Rilonacept

Rilonacept is an IL-1 antagonist containing an IL-1-specific fusion protein, where the IL-1-specific fusion protein contains an IL-1 binding portion of an extracellular domain of human IL-1RAcP, an IL-1 binding portion of an extracellular domain of human IL-1RI, and a multimerizing component. The IL-1-specific fusion protein is described in the US patent applications of US2003/0143697 and US2005/0197293.

Rilonacept, sold under the trade name ARCALYST, is approved by the U.S. Food and Drug Administration (FDA) for treatment of: (1) Cryopyrin-Associated Periodic Syndromes (CAPS), including Familial Cold Autoinflammatory Syndrome (FCAS) and Muckle-Wells Syndrome (MWS) in adults and children aged 2 years and older; (2) DIRA; and (3) recurrent pericarditis (RP).

In addition, rilonacept is also subjected to clinical trials for several diseases, including Gout, Systemic Juvenile Idiopathic Arthritis (SJIA), Schnitzler Syndrome, vascular dysfunction in chronic kidney disease, type 1 diabetes, subacromial bursitis, autoimmune sensorineural hearing loss (ASNHL), cold urticaria, alcoholic hepatitis, arteriosclerosis, and Systemic Sclerosis.

Phase III clinical trials of using rilonacept in the initial stage of uric acid-lowering treatment for gout patients have been completed. Compared with the placebo, rilonacept significantly reduces gout attacks in the initial stage of uric acid-lowering treatment for gout patients.

### Advantages and Disadvantages of Rilonacept

Advantages: Rilonacept can bind to both IL-1α and IL-1β and block signaling pathways mediated by IL-1α and IL-1β, to alleviate inflammatory symptoms of patients. Rilonacept has a longer half-life than Anakinra and only needs weekly injection other than daily injection.

Disadvantages: In addition to IL-1α and IL-1β, Rilonacept also binds to IL-1Ra, which reduces the inhibition efficiency of the IL-1 signaling pathway.

Therefore, drugs that specifically bind to IL-1α and/or IL-1β without binding to IL-1Ra are urgently needed for development.

### SUMMARY OF THE INVENTION

To overcome deficiencies of the prior art, an objective of the present disclosure is to provide a homodimeric protein combining human IL-1R1 with human IL-1RAcP polypeptide fragments, and use thereof. In the present disclosure, the K131E mutation in the human IL-1R1 protein is first identified from a registry of patients with autoinflammatory diseases. This mutation weakens the binding ability of IL-1R1 to IL-1Ra but can ensure normal binding ability of IL-1R1 to IL-1β and/or IL-1α.

In view of this, a technical solution for achieving the technical objective of the present disclosure is as follows:
A human IL-1R1 gene, wherein the human IL-1R1 gene comprises a mutation at a single nucleotide site based on a wild type, in which nucleotides encoding a K131 site are mutated from AAA to GAA, resulting in a K131E mutation in a translated and expressed IL-1R1 protein, and has a gene sequence as shown in SEQ ID No. 1; or wherein the human IL-1R1 gene comprises a mutation at a single nucleotide site based on a wild type, in which nucleotides encoding a K131 site mutated from AAA to GAC, resulting in a K131D mutation in a translated and expressed IL-1R1 protein, and has a gene sequence as shown in SEQ ID No. 2.

The human IL-1R1 gene as described above, wherein the K131E mutation is obtained by screening whole-exome sequencing data of a patient with an autoinflammatory disease, wherein a nucleotide mutation at human genome chromosome 2 locus chr2:102165209, c.391A>G, is identified in the patient, which causes the K131E mutation in the translated human IL-1R1 protein.

A human IL-1R1 protein encoded by the human IL-1R1 gene, having a K131E mutation in which a Lys131 site is mutated to Glu, and an amino acid sequence as shown in SEQ ID NO. 3; or having an artificial mutation based on the K131E mutation, referred to as a K131D mutation, in which a Lys131 site is mutated to Asp, and an amino acid sequence as shown in SEQ ID NO. 4.

The human IL-1R1 protein as described above, which is obtained by protein three-dimensional structure simulation technology, wherein a wild-type K131 site is located at a interaction interface between the IL-1R1 protein and its antagonist IL-1Ra; after the K131 site is mutated to E or D, a surface charge at the K131 site changes from positive to negative, and mutual attraction between a charge at the wild-type K131 site and a charge at an adjacent site on the antagonist IL-1Ra that interacts with the wild-type K131 site is changed to mutual repulsion after the mutation.

The human IL-1R1 protein as described above, wherein in an *in vitro* co-immunoprecipitation assay, the K131E mutation causes weakened interaction between the human IL-1R1 protein and IL-1Ra, but does not affect binding of the IL-1R1 protein to IL-1β and/or IL-1α.

Use of the human IL-1R1 gene as described above in preparation of a medicament, wherein the human IL-1R1 gene is used for preparation of a targeted gene modification therapeutic drug, including an RNA delivery drug, a CAR-T cell gene modification therapeutic drug, and a CRISPR-CAS9 gene modification drug; and the drug is used for treatment of related autoinflammatory diseases clinically diagnosed as caused by the K131E mutation or K131D mutation of the IL-1R1 gene.

Use of the human IL-1R1 protein as described above or a polypeptide fragment thereof in preparation of a medicament, wherein the human IL-1R1 protein or the polypeptide fragment thereof is used for preparation of a protein drug or *in vitro* synthesis of a polypeptide inhibitor, wherein the medicament is used for treatment or prevention of diseases related to overexpression and activation of IL-1α and/or IL-1β, specifically blocking functions of inflammatory activators IL-1α and/or IL-1β while retaining a function of an inflammatory antagonist IL-1Ra, and indications of the medicament include but are not limited to: arthritis, gout, rheumatoid arthritis, Cryopyrin-Associated Periodic Syndromes (CAPS), scleroderma, diabetes, Familial Mediterranean Fever (FMF), Systemic Juvenile Idiopathic Arthritis (SJIA), Periodic Fever, Aphthous Stomatitis, Pharyngitis, and Adenitis Syndrome (PFAPA), Schnitzler syndrome, ankylosing spondylitis, recurrent multifocal osteomyelitis, cancer, dry eye, arteriosclerosis, and uveitis.

A diagnostic kit used for detecting a mutation in a human IL-1R1 gene or a human IL-1R1 protein, wherein the mutation causes a mutation at a translated K131 site, so that a surface charge at the K131 site changes from positive to negative, and mutual attraction between a charge at the wild-type K131 site and a charge at an adjacent site on an antagonist IL-1Ra that interacts with the wild-type K131 site is changed to mutual repulsion after the mutation, wherein the K131 mutation includes a K131E or K131D mutation, and wherein the diagnostic kit includes a nucleic acid amplification kit, a sequencing kit, or an immunoassay kit.

A homodimeric protein specifically binding to IL-1α and/or IL-1β, also referred to as an IL-1 trap protein, which includes two homologous polypeptide sequences, each polypeptide sequence including three segments of amino acid sequences:
a first segment of amino acid sequence including amino acids at positions 21 to 359 of human IL-1RAP, and the first segment of amino acid sequence is as shown in SEQ ID No. 5;
a second segment of amino acid sequence including amino acids at positions 18 to 332 of human IL-1R1, wherein an amino acid at position 131 is mutated from Lys to Glu or Asp, referred to as a K131E or K131D mutation, and the second segment of amino acid sequence is as shown in SEQ ID No. 6 or SEQ ID No. 7; and
a third segment of amino acid sequence including a sequence of human immunoglobulin gamma-1 Fc, and the third segment of amino acid sequence is as shown in SEQ ID No. 8.

The homodimeric protein specifically binding to IL-1α and/or IL-1β as described above, wherein the homodimeric protein having the K131E mutation has a binding activity to human IL-1Ra with an EC50 of 344 ng/mL as determined by enzyme-linked immunosorbent assay.

The homodimeric protein specifically binding to IL-1α and/or IL-1β as described above, wherein the homodimeric protein having the K131E mutation has an affinity constant of 5.069×10⁻⁹ M for IL-1Ra.

The homodimeric protein specifically binding to IL-1α and/or IL-1β as described above, wherein in the presence of IL-1Ra, the IL-1 trap protein having the K131E mutation has a stronger ability to inhibit activation of intracellular NF-κB signaling pathway in human embryonic kidney cell lines in response to IL-1β *in vitro* as compared to an unmutated trap protein.

The homodimeric protein specifically binding to IL-1α and/or IL-1β as described above, wherein the homodimeric protein having the K131E mutation inhibits production of human IL-6 in human lung fibroblasts *in vitro* in response to treatment with human IL-1β, with an IC50 value of 118.2 pM.

The homodimeric protein specifically binding to IL-1α and/or IL-1β as described above, wherein a polypeptide sequence of the homodimeric protein includes an amino acid sequence of SEQ ID No. 9 or SEQ ID No. 10.

An isolated nucleic acid encoding a polypeptide including an amino acid sequence of SEQ ID No. 9 or SEQ ID No. 10.

The nucleic acid as described above, which includes a nucleic acid sequence of SEQ ID No. 11 or SEQ ID No. 12.

Use of the homodimeric protein specifically binding to IL-1α and/or IL-1β as described above in preparation of a medicament, wherein the medicament is used for treatment or prevention of diseases related to overexpression and activation of IL-1α and/or IL-1β, specifically blocking functions of inflammatory activators IL-1α and/or IL-1β while retaining a function of an inflammatory antagonist IL-1Ra, and indications of the medicament include but are not limited to: arthritis, gout, rheumatoid arthritis, Cryopyrin-Associated Periodic Syndromes (CAPS), scleroderma, diabetes, Familial Mediterranean Fever (FMF), Systemic Juvenile Idiopathic Arthritis (SJIA), Periodic Fever, Aphthous Stomatitis, Pharyngitis, and Adenitis Syndrome (PFAPA), Schnitzler syndrome, ankylosing spondylitis, recurrent multifocal osteomyelitis, cancer, dry eye, arteriosclerosis, and uveitis.

A homodimeric protein capable of specifically binding to mouse IL-1α and/or mouse IL-1β, also referred to as a mouse IL-1 trap protein, which includes two homologous polypeptide sequences, each polypeptide sequence including three segments of amino acid sequences:
a first segment of amino acid sequence including amino acids at positions 21 to 359 of mouse IL-1RAP, and the first segment of amino acid sequence is as shown in SEQ ID No. 13;
a second segment of amino acid sequence including amino acids at positions 24 to 336 of mouse IL-1R1, wherein the amino acid at position 134 is mutated from Arg to Glu or Asp, referred to as an R134E or R134D mutation, and the second segment of amino acid sequence is as shown in SEQ ID No. 14 or SEQ ID No. 15; and
a third segment of amino acid sequence including a sequence of mouse immunoglobulin gamma-2A Fc, and the third segment of amino acid sequence is as shown in SEQ ID No. 16.

The homodimeric protein as described above, which inhibits IL-1β-induced mouse IL-6 production in mice, and under the same dosage, the mouse IL-1 trap protein carrying the R134E mutation has a superior inhibitory effect as compared to an unmutated mouse IL-1 trap protein.

The homodimeric protein as described above, which inhibits LPS-induced mouse IL-6 production in mice, and under the same dosage, the mouse IL-1 trap protein carrying the R134E mutation has a superior inhibitory effect as compared to an unmutated mouse IL-1 trap protein.

The homodimeric protein as described above, which alleviates an arthritis phenotype in collagen antibody-induced rheumatoid arthritis model mice, and under the same dosage, the mouse IL-1 trap protein carrying the R134E mutation has a superior inhibitory effect as compared to an unmutated mouse IL-1 trap protein.

The homodimeric protein as described above, wherein a polypeptide sequence of the homodimeric protein includes an amino acid sequence of SEQ ID No. 17 or SEQ ID No. 18.

An isolated nucleic acid encoding a polypeptide including an amino acid sequence of SEQ ID No. 17 or SEQ ID No. 18.

The nucleic acid as described above, which includes a nucleic acid sequence of SEQ ID No. 19 or SEQ ID No. 20.

### Beneficial effects of the present disclosure:

The present disclosure has one or more of the following technical effects:
1. The rare mutation identified according to the present disclosure can be used to guide the preparation of nucleic acid probes or gene diagnostic kits for diagnosing related autoinflammatory diseases.
2. The rare mutation identified according to the present disclosure can be used to guide the development of gene-editing therapies such as CRISPR-Cas9 for the treatment of related autoinflammatory diseases.
3. The gene according to the present disclosure can be used to synthesize polypeptide drugs *in vivo* in the lentiviral delivery therapy.
4. The mutated IL-1R1 gene sequence and the IL-1R1 protein according to the present disclosure can be used for CAR-T therapeutic drugs.
5. The polypeptide dimer protein according to the present disclosure can effectively bind to human IL-1α and/or IL-1β, blocking the binding of IL-1α and IL-1β to the cell membrane receptor IL-1R1.
6. The polypeptide dimer protein according to the present disclosure binds to human IL-1α and/or IL-1β and blocks their binding to the cell membrane receptor IL-1R1, without affecting the effect of IL-1Ra on IL-1R1.
7. Compared with the IL-1 trap protein without mutation, the polypeptide dimer protein according to the present disclosure can more effectively inhibit activation of downstream signaling pathways of IL-1α and/or IL-1β in the presence of IL-1Ra.
8. Compared with common IL-1 inhibitors currently used in clinical practice, the polypeptide dimer protein according to the present disclosure has optimal activity in inhibiting secretion of IL-6 by MRC-5 cells induced by IL-1α and/or IL-1β, and has potential to be prepared into drugs for preventing and/or treating systemic inflammation caused or mediated by high expression of IL-1α and/or IL-1β.
9. The polypeptide dimer protein according to the present disclosure can more rapidly and effectively inhibit the secretion of IL-6 induced by IL-1α and/or IL-1β in mice, as compared to the IL-1 trap protein before mutagenic modification.
10. The polypeptide dimer protein according to the present disclosure can more rapidly and effectively inhibit the production of IL-6 stimulated by bacterial lipopolysaccharide (LPS) in mice, as compared to the IL-1 trap protein before mutagenic modification.
11. The polypeptide dimer protein according to the present disclosure can more effectively inhibit the arthritic phenotype in collagen antibody-induced arthritis model mice, as compared to the IL-1 trap protein before mutagenic modification, and the polypeptide dimer protein has potential to be prepared into drugs for preventing and/or treating a systemic inflammation caused or mediated by high expression of IL-1α and/or IL-1β, especially an arthritis-related disease.

### BRIEF DESCRIPTION OF THE DRAWINGS

FIG. 1 shows the screening for the IL-1R1 gene carrying the K131E mutation from the whole-exome sequencing data of a patient with an autoinflammatory disease.
FIG. 2 shows the Sanger sequencing identification result of the K131E mutation in the IL-1R1 gene in a patient with an autoinflammatory disease.
FIG. 3 shows a schematic diagram of structural simulation of the interaction between IL-1R1 protein and IL-1Ra protein.
FIG. 4 shows the co-immunoprecipitation detection results of K131E mutant and control wild-type IL-1R1 proteins with human IL-1Ra and IL-1β.
FIG. 5 shows a schematic diagram of surface charge simulation of the structures of control wild type and K131 site-directed mutant IL-1R1 proteins.
FIG. 6 shows the co-immunoprecipitation detection results of control wild-type and K131 site-directed mutant IL-1R1 proteins with human IL-1Ra.
FIG. 7A shows the structures and compositions of control wild-type and K131E mutant IL-1 trap proteins.
FIG. 7B shows the detection results of the binding activity of K131E mutant and control wild-type IL-1 trap proteins to human IL-1α/IL-1β/IL-1Ra.
FIG. 8 shows the determination results of the affinity constants of K131E mutant and control wild-type IL-1 trap proteins with human IL-1Ra.
FIG. 9 shows the detection results of the blocking of IL-1β-activated NF-κB signaling pathway by K131E mutant and control wild-type IL-1 trap proteins.
FIG. 10 shows the detection results of the inhibitory characterization of K131E mutant, control wild-type IL-1 trap proteins, and common IL-1 inhibitors on IL-1β-induced IL-6 production in human cells.
FIG. 11A shows the murine R134E mutation.
FIG. 11B shows the detection results of the inhibitory characterization of the control wild-type IL-1 trap protein on IL-1β-induced IL-6 production in mice.
FIG. 12 shows the detection results of the inhibitory characterization of the murine R134E mutant and control wild-type IL-1 trap proteins on LPS-induced IL-6 production in mice.
FIG. 13A shows representative photographs of paws from different dosing groups in the experiment where murine R134E mutant and control wild-type IL-1 trap proteins alleviate the arthritic phenotype in collagen antibody-induced arthritis model mice.
FIG. 13B shows the changes in arthritis index of mice in different groups in the experiment where murine R134E mutant and control wild-type IL-1 trap proteins alleviate the arthritic phenotype in collagen antibody-induced arthritis model mice.
FIG. 13C shows representative CT photographs of paws from different groups of mice in the experiment where murine R134E mutant and control wild-type IL-1 trap proteins alleviate the arthritic phenotype in collagen antibody-induced arthritis model mice.

### DETAILED DESCRIPTION

The present disclosure is further described below with reference to accompanying drawings and examples.

### Example 1

### Screening for the IL-1R1 gene carrying the K131E mutation from the whole-exome sequencing data of a patient with an autoinflammatory disease

Main materials, equipment and software: Peripheral blood of the patient and his/her immediate family members, Maxwell RSC automated nucleic acid purification platform and kit (Promega), Covaris instrument, Axygen^{®} AxyPrep MAG PCR Clean-Up Kit, Agilent SureSelect Hybridization and Wash Kits, Illumina sequencer, Samtools (1.7), BWA (0.7.17-r1188), Picard (2.25.4), GATK (4.2.0.0), and Annovar (2019Oct24).

Operation method: The operations can be divided into three parts: DNA extraction, DNA library construction and sequencing, and whole-exome data analysis.

DNA extraction: First, DNA was extracted from peripheral blood of the patient and his/her parents via the Maxwell RSC automated nucleic acid purification platform and kit (Promega), and then whole exome library construction and sequencing were performed on the DNA of the patient and his/her parents.

**The steps of DNA library construction and sequencing are as follows:**
(1) DNA sample testing. Sample testing includes measurement of a concentration, integrity and purity of samples. The concentration was measured by Nanodrop 2000. The integrity and purity of DNA samples were measured by agarose gel electrophoresis.
(2) 1 µg of genomic DNA was taken and ultrasonically fragmented to 250-300 bp via a Covaris instrument. After fragmentation, a fragmentation effect on the DNA was first evaluated by agarose gel electrophoresis, and then purified using Axygen^{®} AxyPrep MAG PCR Clean-Up Kit.
(3) End repair. The end repair reaction system was prepared, and after allowing the reaction to proceed for a certain period of time, purification was performed using the AxyPrep Mag PCR Clean-Up Kit.
(4) End addition of "A" and adapter ligation. The end "A" addition reaction system and the adapter ligation reaction system were prepared, and incubated at room temperature for a certain period. The product was purified using the AxyPrep Mag PCR Clean-Up Kit.
(5) Pre-PCR reaction and product recovery. The PCR reaction system was prepared, the reaction program was set, and purification was performed using the AxyPrep Mag PCR Clean-Up Kit.
(6) A certain amount of PCR product was taken for hybridization and subjected to hybridization and elution via Agilent SureSelect Hybridization and Wash Kits.
(7) PCR reaction and product recovery. The PCR reaction system was prepared, the reaction program was set, and the product obtained after hybridization and elution was amplified. The product was purified using the AxyPrep Mag PCR Clean-Up Kit.
(8) The constructed library was tested for quality and yield via the Agilent 2100 Bioanalyzer and ABI SteponePlus Real-Time PCR System.
(9) A qualified library was sequenced on the Illumina sequencing platform.

### Whole-exome data analysis:

(1) Quality control of sequencing data was performed.
(2) The sequencing data was aligned to the human reference genome (Homo_sapiens_assembly38) using BWA to obtain the initial alignment file (SAM file).
(3) The file obtained in the previous step was sorted and converted in format using Samtools, and PCR duplicates were removed using Picard.
(4) Variants in the sequencing data were identified using GATK, and VCF files from the same family were merged.
(5) The obtained variant information was annotated using ANNOVAR.
(6) Genetic analysis of the obtained variants was conducted within the family, and low-frequency, non-synonymous variants conforming to the law of segregation were identified as candidates.

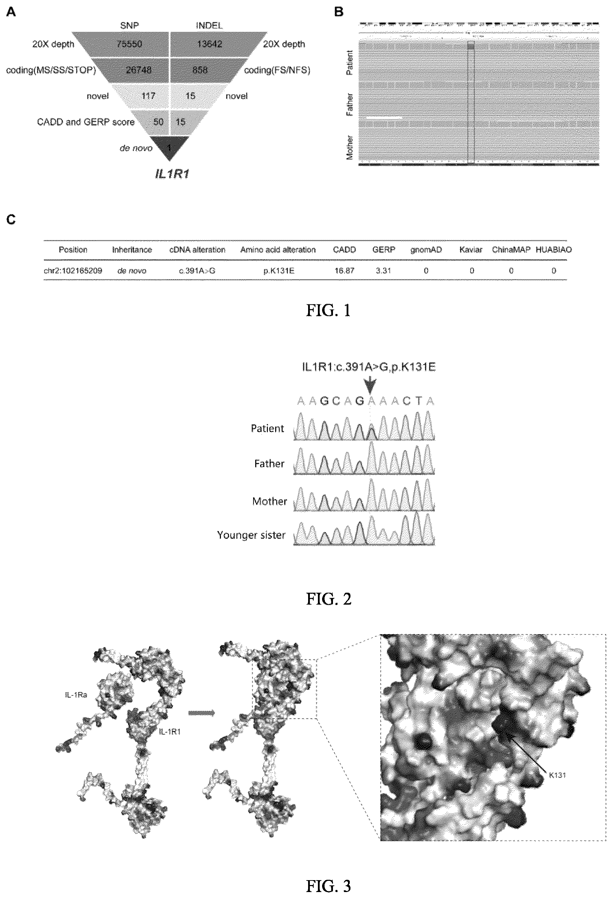

FIG. 1 shows the screening strategy, process, and results of the IL-1R1 gene with K131E mutation from the whole-exome sequencing data of the patient with an autoinflammatory disease. FIG. 1 includes parts (A), (B), and (C). Part (A) describes the process of step-by-step filtering and screening of mutations using a Python program after obtaining mutations (SNPs and INDELs) in the patient and his/her family members using the GATK bioinformatics algorithm. From top to bottom, mutations are filtered sequentially to retain mutations with a coverage depth of no less than 20X, mutations affecting coding, mutations not recorded in mutation databases, mutations that are conservative and predicted to be deleterious, and newly emerged mutations. Part (B) shows the IGV diagram of the alignment results of the whole-exome sequencing data of the patient and his/her parents at the mutation site. Part (C) shows the base variation information of the mutation site, indicating a nucleotide mutation at chr2:102165209 site on human chromosome 2, c.391A>G, i.e. a K131E mutation occurred in the IL-1R1 protein.

### Example 2

### Sanger sequencing results of the K131 sites in the IL-1R1 genes in a patient diagnosed with a new type of autoinflammatory disease and his/her healthy immediate family members

Main materials: Blood from the patient and their immediate family members, Maxwell RSC automated nucleic acid purification platform and kit (Promega), KOD FX PCR reagents (TOYOBO).

Operational method: After approval from the hospital ethics committee and informed consent forms signed by the patient and his/her immediate family members were obtained, 1 mL of venous blood from the patient and his/her immediate family members was drawn into heparin anticoagulant tubes. The blood was transferred into the kit according to the instruction for use of the Maxwell RSC automated nucleic acid purification platform to obtain purified genomic DNA. Primer pairs were designed at approximately 200 bp upstream and downstream of the mutation site (upstream primer: CGTGGTAAGGTAAGAGAGGAATTAG; and downstream primer: AGGTTCCCTATCAAGTTTCACC). Genomic DNAs of the patient and his/her immediate family members were used as templates, to perform PCR experiments according to the instructions for use of the KOD FX PCR kit. After the amplified DNA sequences were subjected to DNA agarose gel electrophoresis and stained with green as blue nucleic acid dye (UV type) to determine PCR products of a correct size, DNA bands were cut, and DNA fragments were recovered with a nucleic acid recovery kit (MN, Germany), to perform Sanger sequencing. The sequencing results were analyzed and aligned via SnapGene software.

The results in FIG. 2 show the Sanger sequencing results of the gene sequence at and near the mutation site in the patient carrying the IL-1R1 mutation and his/her immediate family members. A double peak in sequencing can be observed at the patient's mutation site, where the high peak indicates nucleotide A and the low peak indicates nucleotide G. That is, an A/G double peak appears at the patient's mutation site, revealing that the patient has a heterozygous mutation at this gene locus; in contrast, the sequencing result of this locus in the patient's immediate family members shows a single A peak. The results in FIG. 2 confirm the c.391A>G mutation identified in Example 1.

### Example 3

### Structure of the wild-type IL-1R1 protein interacting with IL-1R1 protein, and protein surface net charge stimulation

Main materials and software: Pymol software, the protein complex structure of IL-1R1 and IL-1Ra during interaction (PDB code: 1IRA), and the protein structure prediction website AlphaFold.

Operational method: Pymol software was used to unfold the protein complex structure of IL-1R1 and IL-1Ra during interaction (PDB code: 1IRA), and the structures of individual IL-1R1 and IL-1Ra predicted by AlphaFold were unfolded. These three protein structures were aligned and superimposed in the software, and then the protein complex structure of IL-1R1 and IL-1Ra during interaction was hidden. This yielded clear independent structures of IL-1R1 and IL-1Ra in their interacting state. The independent structures of IL-1R1 and IL-1Ra were annotated with electrostatic potential using PyMOL software: positive charges were labeled in blue (darker in grayscale images) and negative charges in red (lighter in grayscale images).

FIG. 3 shows the simulated protein structures of IL-1R1 and IL-1Ra. Through electrostatic simulation, the blue mark indicated positive charges on the protein surface, and the red mark indicated negative charges on the protein surface. The right part of FIG. 3 is a local enlarged view of an interaction interface between IL-1R1 and IL-1Ra. It can be seen that the K131 site was located at the interaction interface between IL-1R1 and IL-1Ra, carried an obvious positive charge, and was surrounded by negatively charged polypeptide groups of IL-1Ra.

### Example 4

### Co-immunoprecipitation detection results of K131E mutant and control wild-type IL-1R1 proteins with human IL-1Ra and IL-1β

Main materials: HEK293T cell line, Flag-tagged K131E mutant and control wild-type IL-1R1 protein expression plasmids, recombinant human IL-1β/IL-1Ra protein (Sino Biological), and anti-Flag antibody conjugated to magnetic beads (Biomake).

Operational method: HEK293T cell line was cultured in a 6 cm petri dish and transfected with Flag-tagged K131E mutant and control wild-type IL-1R1 proteins, respectively. After 24 hours of protein expression, the cell culture medium was replaced with a medium containing 0.5 µg/ml IL-1β, and the cells were further cultured for one hour. 200 microliters of medium was collected from each petri dish and mixed with 4× loading buffer, and then the resulting mixture was heated at 95°C for 10 minutes for later use. The remaining medium was discarded, and adherent cells were washed with PBS. After PBS was aspirated and discarded, 250 µL of 1% Triton X-100 cell lysis buffer (20 mM Tris-HCl, pH 7.5, 1% Triton X-100, 150 mM NaCl, 10% glycerol, 2 mM EDTA, and protease inhibitors) was added to each dish of cells. The cells were scraped off with a cell scraper and pipetted, the cell lysate was aspirated and transferred to a 1.5 mL EP tube, and the resulting mixture was mixed for lysis at 4°C for 45 minutes. The lysed cells were centrifuged at 13000g for 15 minutes, and the supernatant was aspirated. 20 µL of anti-Flag antibody conjugated to magnetic beads was added to each tube of cell lysate. After the resulting mixture was rotated and incubated in a 4°C refrigerator for 4 hours, the magnetic beads were washed by pipetting with the lysis buffer 4 times, and washed with pre-cooled PBS for the last time. An appropriate amount of 2× loading buffer was added to the magnetic beads, ensuring full contact with the magnetic beads. The resulting mixture was centrifuged for a suitable period to spin down, to the bottom of the tube, liquid adhering to a tube wall. The resulting mixture was heated at 95°C for 10 minutes and then centrifuged. 20 µL of the supernatant was taken for loading and detected by Western blotting.

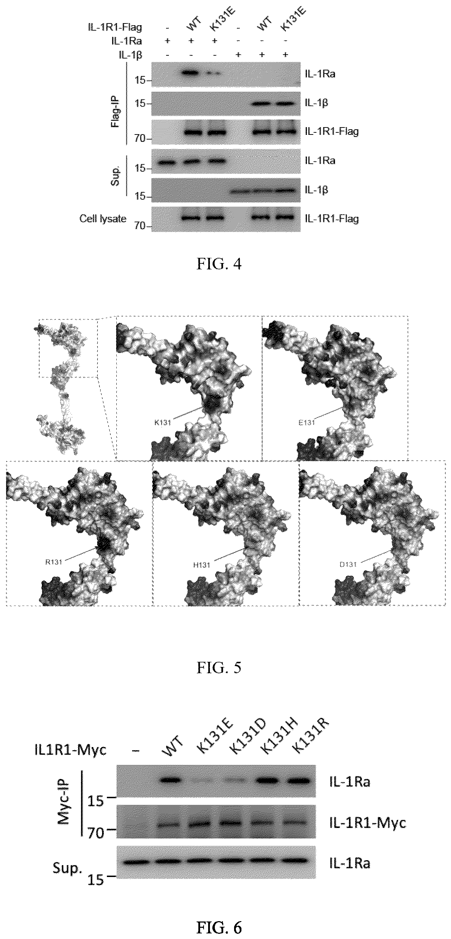

In FIG. 4, the first three loading wells indicate the interaction between wild-type and K131E mutant IL-1R1 with IL-1Ra. The first well served as a negative control to exclude non-specific binding of magnetic beads; and the second and third loading wells were used for co-immunoprecipitation of IL-1Ra with wild-type and K131E mutant IL-1R1, respectively. As shown in the data of the first row, the K131E mutation impaired the interaction between IL-1R1 and IL-1Ra. The fourth to sixth loading wells in FIG. 4 indicate the interaction between wild-type and K131E mutant IL-1R1 with IL-1β. The fourth well served as a negative control to exclude non-specific binding of magnetic beads; and the fifth and sixth loading wells were used for co-immunoprecipitation of IL-1β with wild-type and K131E mutant IL-1R1, respectively. According to the data of the second row, the K131E mutation did not alter the interaction between IL-1R1 and IL-1β

### Example 5

### Simulation of surface net charge of the structures of control wild type and K131 site-directed mutant IL-1R1 proteins

Main materials and software: Pymol software and IL-1R1 protein structure predicted by AlphaFold.

Operational method: The structure of IL-1R1 was unfolded using Pymol software. The protein surface electrostatic potential of IL-1R1 was annotated: positive charges were labeled in blue (darker in grayscale images) and negative charges in red (lighter in grayscale images). Subsequently, the lysine at position 131 was mutated to glutamic acid (K131E), aspartic acid (K131D), arginine (K131R), and histidine (K131H), respectively, and the protein surface electrostatic potential of each mutant was annotated separately.

FIG. 5 shows the schematic diagram of the protein surface charge simulation of IL-1R1 when the K131 site of wild-type IL-1R1 (K131) was site-directedly mutated to amino acids with different charges. When K131 was mutated to arginine (K131R) or histidine (K131H), the protein surface charge at site 131 remained blue (darker in the grayscale image), indicating a positive charge. When K131 was mutated to negatively charged amino acids such as glutamic acid (K131E) or aspartic acid (K131D), the protein surface charge changed to red (lighter in the grayscale image), indicating a negative charge.

### Example 6

### Co-immunoprecipitation detection results of control wild type and K131 site-directed mutant with human IL-1Ra

Main materials: HEK293T cell line, expression plasmids of Myc-tagged K131E, K131D, K131R, K131H mutants and control wild-type IL-1R1 proteins, recombinant human IL-1Ra protein (Sino Biological), and anti-Myc antibody conjugated to magnetic beads (Biomake).

Operational method: HEK293T cell line was cultured in a 6 cm petri dish and transfected with Myc-tagged K131E, K131D, K131R, K131H mutants and control wild-type IL-1R1 proteins, respectively. After 24 hours of protein expression, the cell culture medium was replaced with a medium containing 0.5 µg/ml IL-1Ra, and the cells were further cultured for one hour. 200 microliters of medium was collected from each petri dish and mixed with 4× loading buffer, and then the resulting mixture was heated at 95°C for 10 minutes for later use. The remaining medium was discarded, and adherent cells were washed with PBS. After PBS was aspirated and discarded, 250 µL of 1% Triton X-100 cell lysis buffer (20 mM Tris-HCl, pH 7.5, 1% Triton X-100, 150 mM NaCl, 10% glycerol, 2 mM EDTA, and protease inhibitors) was added to each dish of cells. The cells were scraped off with a cell scraper and pipetted, the cell lysate was aspirated and transferred to a 1.5 mL EP tube, and the resulting mixture was mixed for lysis at 4°C for 45 minutes. The lysed cells were centrifuged at 13000g for 15 minutes, and the supernatant was aspirated. 20 µL of anti-Myc antibody conjugated to magnetic beads was added to each tube of cell lysate. After the resulting mixture was rotated and incubated in a 4°C refrigerator for 4 hours, the magnetic beads were washed by pipetting with the lysis buffer 4 times, and washed with pre-cooled PBS for the last time. An appropriate amount of 2× loading buffer was added to the magnetic beads, ensuring full contact with the magnetic beads. The resulting mixture was centrifuged for a suitable period to spin down, to the bottom of the tube, liquid adhering to the tube wall. The resulting mixture was heated at 95°C for 10 minutes and then centrifuged. 20 µL of the supernatant was taken for loading and detected by Western blotting.

FIG. 6 shows the interaction between IL-1R1 and IL-1Ra after site-directed mutations with different charges occurred at the K131 site of the wild-type IL-1. The first well served as a negative control to exclude non-specific binding of magnetic beads; the second to sixth loading wells were used for co-immunoprecipitation of IL-1Ra with wild-type IL-1R1 and K131E, K131D, K131R, K131H mutants, respectively. As shown in the data of the first row, the K131E and K131D mutations, which cause charge reversal at K131, impaired the interaction between IL-1R1 and IL-1Ra, whereas mutations that did not alter the charge had no effect on the interaction between IL-1R1 and IL-1Ra.

### Example 7

### Study on the binding activity of the K131E mutant according to the present disclosure and control wild-type IL-1 trap proteins to human IL-1α/IL-1β/IL-1Ra (ELISA method) (receptor binding assay)

Main materials: IL-1 trap proteins of K131E mutant according to the present disclosure and the control wild type, recombinant human IL-1α/IL-1β/IL-1Ra proteins (Sino Biological), and Duoset ELISA kit (R&D Systems).

### Operational method:

The binding ability of K131E mutant and control wild-type IL-1 trap proteins to human IL-1α/IL-1β/IL-1Ra was detected by receptor binding assay. First, the recombinant human IL-1α/β/Ra was diluted to the working concentration (10 µg/mL) with PBS, and 100 µL was immediately used for coating each well in a 96-well plate. The plate was sealed and allowed to be coated overnight at 4°C. The liquid in the wells was discarded and the plate was washed with washing buffer (0.05% Tween 20 in PBS, pH 7.2-7.4). Each well was blocked with 300 µL of diluent (PBS containing 1% BSA, pH 7.2-7.4, filtered through 0.2 µm) and allowed to be blocked at room temperature for 1 hour. 100 µL of samples (recombinant IL-1 trap proteins WT/K131E-Fc) in reagent diluent was added to each well (with concentration gradients ranging from 1 ng/mL to 10000 ng/mL). The plate was covered with a sealing film and incubated at room temperature for 2 hours. 100 µL of detection antibody (goat anti-human IgG-Fc secondary antibody-HRP) (Sino Biological) (0.05 µg/mL) was added and diluted in the reagent diluent, and the resulting mixture was added to each well. The plate was covered with a new sealing film and incubated at room temperature for 2 hours. The resulting mixture was repeatedly aspirated/washed with the washing buffer. 100 µL of reaction solution was added to each well and the plate was incubated at room temperature for 20 minutes in the dark. 50 µL of stop solution was added to each well. The plate was gently tapped to ensure thorough mixing. A microplate reader set to 450 nm was used to immediately measure an optical density of each well. A correction wavelength was set to 540 nm, the readings were recorded, and the readings at 540 nm were subtracted from the readings at 450 nm. Finally, Prism 8 software was used to calculate a median effective concentration (EC₅₀) of the two IL-1 trap proteins.

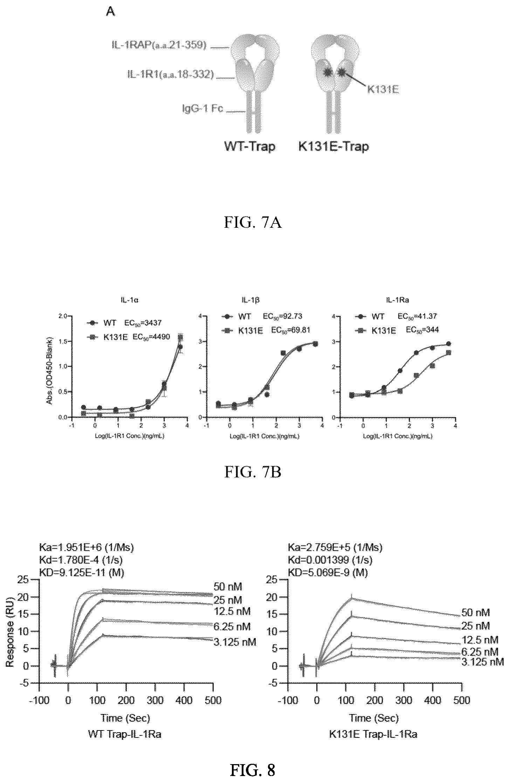

FIG. 7A shows the structures and compositions of the control wild-type and K131E mutant IL-1 trap proteins. Both have a main structure containing two homologous polypeptide sequences, and each polypeptide sequence includes three segments of amino acid sequences. The first segment of amino acid sequence includes amino acids at positions 21 to 359 of human IL-1RAP; the second segment of amino acid sequence includes amino acids at positions 18 to 332 of human IL-1R1, where the amino acid at position 131 in the sequence of the IL-1 trap protein with the K131E mutation is mutated from Lys to Glu, i.e., a K131E mutation; and the third segment of amino acid sequence includes a sequence of human immunoglobulin gamma-1 Fc. FIG. 7B shows the signal intensity corresponding to the amount of IL-1 trap protein bound to IL-1α (left), IL-1β (middle), and IL-1Ra (right) as the concentration of wild-type (dots) and K131E mutant (squares) IL-1 trap proteins (Trap) increases. As shown in the figure, the K131E mutant IL-1 trap protein shows significantly reduced binding to IL-1Ra compared to the unmutated wild-type (WT) trap protein. The EC50 value of the wild-type trap protein for binding to IL-1Ra is 41.37 ng/ml; the EC50 value of the K131E mutant trap protein for binding to IL-1Ra is 344 ng/ml. However, the K131E mutation has no significant effect on the binding of the trap protein to IL-1α/1L-1β.

### Example 8

### Determination of the affinity constant of the K131E mutant IL-1 trap protein according to the present disclosure and control wild-type IL-1 trap protein with human IL-1Ra

Main materials: IL-1 trap proteins of the K131E mutant according to the present disclosure and the control wild type, recombinant human IL-1Ra protein (Sino Biological), HBS-EP+, Protein A chip (Cytiva), reproduction solution Glycine 1.5, deionized water, uncovered 1.5 mL EP tubes, and Biacore T200 molecular interaction instrument.

### Operational method:

The affinity constants between K131E mutant/control wild-type IL-1 trap proteins and human IL-1Ra were determined using a Biacore molecular interaction instrument. 50 mL of 10× HBS-EP and buffer, and 450 mL of deionized water (filtered through a 0.22 µm membrane) were taken and mixed thoroughly, and then the resulting mixture was transferred to a 500 mL buffer bottle. A buffer inlet tube and a waste liquid tube were connected to the bottle. The chip was inserted and a pipeline was cleaned via a program. Preparation of ligands (IL-1 trap proteins of the K131E mutant and the control wild type): The IL-1 trap proteins were diluted to 1 µg/mL with 1 x HBS-EP and buffer. Analyte (IL-1Ra) concentrations: IL-1Ra was diluted to 0, 0.78, 1.56, 3.125, 6.25, 12.5, 25, 50, 100, and 200 nM with 1 x HBS-EP and buffer. Kinetics/Affinity program and parameters (ligand binding time of 60 seconds, sample binding time of 120 seconds, a flow rate of 30 µL/min, time of 30s, dissociation time of 600 seconds, a reproduction reagent of Glycine 1.5, and reproduction time of 30 seconds) were set. Corresponding samples were prepared as content displayed on the screen and placed at designated positions. Data collection was performed via Biacore Control 2.0 software, and data analysis was conducted via Biacore T200 Evaluation 2.0 software.

FIG. 8 shows binding the binding reactions detected by the Biacore instrument between K131E mutant/control wild-type IL-1 trap proteins and IL-1Ra at different concentrations. The data shows that an affinity constant of the wild-type trap protein for IL-1Ra is 9.125×10⁻¹¹ M, and an affinity constant of the K131E mutant trap protein for IL-1Ra is 5.069×10⁻⁹ M. Ka indicates an association constant, and Kd indicates a dissociation constant. The data shows that compared with the wild-type trap protein, the K131E mutant trap protein has a smaller association constant (Ka) and a larger dissociation constant (Kd) for IL-1Ra.

### Example 9

### Blocking of IL-1β-activated NF-κB signaling pathway by K131E mutant IL-1 trap protein according to the present disclosure and control wild-type IL-1 trap protein

Materials: IL-1 trap proteins of K131E mutant according to the present disclosure and the control wild type, HEK293T cells, NF-κB dual-luciferase reporter gene, luciferase reporter gene assay kit (Promega), and recombinant human IL-1α/IL-1β/IL-1Ra proteins (Sino Biological).

### Operational method:

The neutralizing biological activity of the K131E mutant and control wild-type IL-1 trap proteins in blocking L-1β-activated NF-κB signaling pathway (with/without IL-1Ra addition) was detected using the fluorescent reporter gene method. 293T cells were routinely digested and seeded in a 96-well plate at a density of 5000 cells per well, and 24 hours after the cells adhered to the wall, the cells were transfected with overexpressed dual-luciferase reporter gene. After 24 hours, the cells were divided into 6 groups for drug treatment: 1. Blank control group (no drug added); 2. IL-1β group (1 ng/mL); 3. IL-1β + WT Trap group (1 ng/mL IL-1β + 1 nM WT Trap); 4. IL-1β + K131E Trap group (1 ng/mL IL-1β + 1 nM K131E Trap); 5. IL-1β + IL-1Ra + WT Trap group (1 ng/mL IL-1β + 50 ng/mL IL-1Ra + 1 nM WT Trap); and 6. IL-1β + IL-1Ra + K131E Trap group (1 ng/mL IL-1β + 50 ng/mL IL-1Ra + 1 nM K131E Trap). The drugs for groups 3, 4, 5, and 6 were pre-mixed at 37°C for 2 hours before cell treatment. After 6 hours of co-cultivation with drugs, the supernatant was removed, PBS was added to each well for washing once, and the washing solution was discarded. 50 µL of PLB lysis buffer was added to each well, and the plate was put on a shaker for lysis at room temperature for 30 minutes. 20 µL of the lysate was pipetted from each well into a 96-well assay plate, 50 µL of LARII solution was added, and the plate was put into a fluorescence detector (during measurement, a time delay of 1-2 seconds and a reading time of 5 seconds were used) to detect intensity of luciferin. Subsequently, 50 µL of Stop&Glo solution was added to detect intensity of Renilla luciferase. For data processing, the fluorescence data of the experimental groups was compared with the fluorescence value of the internal reference Renilla luciferase to obtain the relative activity of the reporter gene.

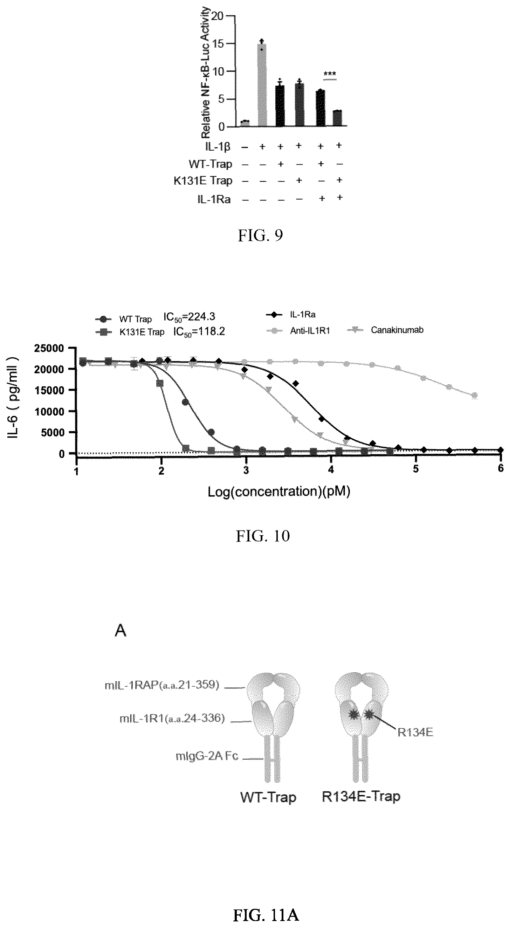

In FIG. 9, starting from the left side, the first column indicates the NF-κB transcriptional activity of the control cells, and the second column indicates that IL-1β activates the intracellular NF-κB transcriptional activity; the third and fourth columns respectively indicate that in the absence of IL-1Ra, the control wild-type and K131E mutant IL-1 trap proteins have consistent efficiencies in blocking the IL-1β-activated NF-κB signaling pathway; and the fifth and sixth columns respectively show the efficiency of the control wild-type and K131E mutant IL-1 trap proteins in blocking the IL-β-activated NF-κB signaling pathway in the presence of IL-1Ra, where the results indicate that the K131E mutant IL-1 trap protein has a stronger blocking and inhibitory capability.

### Example 10

### Characterization of the inhibitory effect of the K131E mutant IL-1 trap protein according to the present disclosure and control wild-type IL-1 trap protein, in comparison with a common IL-1 inhibitor, on IL-1β-induced IL-6 production in human cells

Materials: IL-1 trap proteins of a K131E mutant according to the present disclosure and control wild type, MRC-5 cells, human IL-6 ELISA kit (Multi Sciences (Lianke) Biotech), recombinant human IL-1β (Sino Biological), recombinant human IL-1Ra protein (Sino Biological), anti-IL-1R1 monoclonal antibody (R&D Systems), and anti-IL-1β monoclonal antibody/Canakinumab (Novartis Pharmaceuticals).

### Operational method:

The anti-IL-1β activity of different inhibitors *in vitro* was determined by detecting their ability to inhibit IL-1β-induced IL-6 expression and secretion in human MRC-5 cells. Human MRC-5 cells (purchased from Mingzhoubio) were routinely digested, counted, and then seeded into a flat-bottom 96-well plate at a density of 3000 cells per well, and the plate was put in a cell incubator for cultivation. After 24 hours (when the cells reached 80% confluence), the medium was replaced with fresh medium, and the cells were cultured for 10 minutes.

Cells were divided into 5 groups and treated with drugs (drugs in each group were pre-mixed at 37°C for 2 hours, with all final concentrations of IL-1β being 1 ng/mL):
1. IL-1β + WT Trap group: WT Trap was set at 13 concentrations (starting concentration 50,000 pM, 2-fold serial dilution down to 12.21 pM);
2. IL-1β + K131E Trap group: K131E Trap was set at 13 concentrations (starting concentration 50,000 pM, 2-fold serial dilution down to 12.21 pM);
3. IL-1β + Canakinumab group: Canakinumab was set at 13 concentrations (starting concentration 30,000 pM, 2-fold serial dilution down to 7.32 pM);
4. IL-1β + IL-1Ra group: IL-1Ra was set at 13 concentrations (starting concentration 100,000 pM, 2-fold serial dilution down to 244.14 pM);
5. IL-1β + anti-IL-1R1 monoclonal antibody group: anti-IL-1R1 monoclonal antibody was set at 13 concentrations (starting concentration 100,000 pM, 2-fold serial dilution down to 244.14 pM).
After the drugs were added to the cells, the cells were placed in a cell incubator for culture for 24 hours. The cell supernatant was collected and detected using a human IL-6 ELISA kit.

FIG. 10 shows the detection results of the inhibitory characterization of the K131E mutant and control wild-type IL-1 trap proteins, in comparison with a common IL-1 inhibitor, on IL-1β-induced IL-6 production in human cells. FIG. 10 shows the ability of the K131E mutant and control wild-type IL-1 trap proteins, and the common IL-1 inhibitor to inhibit IL-6 production in human lung fibroblasts in response to IL-1β at different drug concentrations. Among them, the K131E mutant IL-1 trap protein exhibited the strongest ability to inhibit IL-1β compared to the wild-type IL-1 trap protein and the common IL-1 inhibitor, with an IC50 value of 118.2 pM for its inhibitory effect.

### Example 11

### Inhibitory characterization of the R134E mutant murine IL-1 trap protein according to the present disclosure and control wild-type IL-1 trap protein on IL-1β-induced IL-6 production in mice

Materials: IL-1 trap proteins of murine R134E mutant according to the present disclosure and control wild type, 8-week-old female C57BL/6 mice, recombinant murine IL-1β (MCE), and mIL-6 ELISA kit (Multi Sciences (Lianke) Biotech).

### Operational method:

The ability of the murine R134E mutant and control wild-type IL-1 trap proteins to inhibit IL-1β *in vivo* was evaluated by detecting their effect on mIL-1β-induced IL-6 production levels in mice. It has been reported that due to species differences, human IL-1 trap proteins have weak binding to murine IL-1β and IL-1Ra. The inventors redesigned and synthesized murine IL-1 trap proteins (R134E mutant and control wild-type murine IL-1 trap proteins) that are conserved with respect to the human IL-1 trap protein. Female C57BL/6 mice were divided into 6 groups with 8 mice in each group, and administered via intraperitoneal injection. 1. R134E mutant murine IL-1 trap protein (mIL-1 Trap) (10-fold molar amount of mIL-1β, 1.25 µg per mouse); 2. R134E mutant murine IL-1 trap protein (mIL-1 Trap) (50-fold molar amount of mIL-1β, 6.25 µg per mouse); 3. wild-type murine IL-1 trap protein (mIL-1 Trap) (10-fold molar amount of mIL-1β, 1.25 µg per mouse); 4. wild-type murine IL-1 trap protein (mIL-1 Trap) (50-fold molar amount of mIL-1β, 6.25 µg per mouse); 5. injection of an equal volume of PBS; and 6. injection of an equal volume of PBS.

After 1 hour, mice in groups 1-5 were injected with mIL-1β (0.5 µg/kg); and mice in group 6 were injected with an equal volume of PBS. Blood samples were collected at 2 hours, plasma was collected, and the level of mIL-6 in the plasma was analyzed by ELISA.

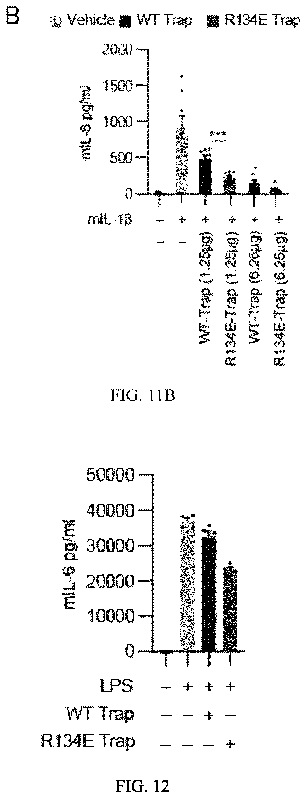

The detection results of inhibition characterization of IL-1 β-induced IL-6 production by IL-1 trap proteins of murine R134E mutant (shown in FIG. 11A) and control wild type (shown in FIG. 11B) in mice are shown. The results show that the first column from the left indicates that the plasma IL-6 level in control mice is very low, close to 0; the second column indicates that the plasma mIL-6 concentration in mice increases rapidly after injection of murine IL-1β; the third (WT-Trap) and fourth (R134E-Trap) columns respectively indicate the mIL-6 concentrations when the injection dose is 1.25 µg per mouse; the fifth (WT-Trap) and sixth (R134E-Trap) columns respectively indicate the mIL-6 concentrations when the injection dose is 6.25 µg per mouse. The results show that at both tested concentrations, the R134E mutant murine IL-1 trap protein exhibits a stronger inhibitory effect on IL-1β-induced IL-6 production in mice compared to the wild-type IL-1 trap protein.

### Example 12

### Inhibitory characterization of the R134E mutant IL-1 trap protein according to the present disclosure and control wild-type IL-1 trap protein on LPS-induced IL-6 production in mice

Materials: IL-1 trap proteins of murine R134E mutant according to the present disclosure and control wild type, 8-week-old female BALB/c mice, lipopolysaccharide (LPS) (MCE), and mIL-6 ELISA kit (Multi Sciences (Lianke) Biotech).

### Operational method:

The ability of murine R134E mutant and control wild-type IL-1 trap proteins to inhibit inflammatory responses caused by simulated bacterial infection in mice was evaluated by detecting their effect on LPS-induced IL-6 production levels in mice. Female BALB/c mice were divided into 4 groups with 5 mice in each group, and administered via intraperitoneal injection. 1. R134E mutant murine IL-1 trap protein (mIL-1 Trap) (10 mg/kg) + LPS (25 µg per mouse); 2. wild-type murine IL-1 trap protein (mIL-1 Trap) (10 mg/kg) + LPS (25 µg per mouse); 3. LPS (25 µg per mouse); and 4. control group, injected with an equal volume of PBS.

LPS was injected 24 hours after the injection of the IL-1 trap protein. Blood samples were collected from the mice 4 hours after LPS administration, plasma was collected, and the level of mIL-6 in the plasma was analyzed by ELISA.

As shown in Figure 12, the results demonstrate the inhibitory characterization of murine R134E mutant and control wild-type IL-1 trap proteins on LPS-induced IL-6 production in mice. The first column from the left indicates that the plasma IL-6 level in control mice is very low; the data in the second column (Vehicle) confirm that LPS injection rapidly increases the plasma IL-6 expression in mice; a comparison between the third column (WT Trap) and the fourth column (R134E Trap) shows that the murine R134E mutant IL-1 trap protein exhibits a stronger inhibitory effect on LPS-induced IL-6 production in mice compared to the wild-type IL-1 trap protein.

### Example 13

### The R134E mutant IL-1 trap protein according to the present disclosure and control wild-type IL-1 trap protein alleviate the arthritis phenotype in collagen antibody-induced arthritis (CAIA) model mice

Materials: IL-1 trap proteins of murine R134E mutant according to the present disclosure and control wild type, Balb/c mice, anti-collagen antibody mixture (Chondrex), lipopolysaccharide LPS (Chondrex), and high-resolution micro-CT scanner (U-CT-XUHR, Milabs).

### Operational method:

Seven-to-eight-week-old Balb/c mice were intraperitoneally injected with 1.5 mg of anti-collagen antibody mixture per mouse. Three days after the injection, the mice were intraperitoneally injected with LPS (25 µg per mouse) to induce arthritis. 2, 5 and 8 days after the injection of the anti-collagen antibody, the mice were intraperitoneally injected with IL-1 trap proteins of the murine R134E mutant and control wild type at a dose of 10 mg/kg, or an equal volume of PBS. Based on symptoms of swelling and inflammation, clinical arthritis of the mice was scored daily before and after drug injection, with a scoring range of 0 to 16 points. There were 5 mice in each group. Scoring criteria: Each limb was scored as follows: 0. normal, with no visible symptoms of arthritis; 1. mild redness and swelling of the ankle, or obvious redness and swelling of only one finger, regardless of the number of affected fingers; 2. moderate redness and swelling of the ankle joint; 3. redness and swelling of the entire paw including the fingers; and 4. limb stiffness involving multiple joints, affecting movement (the maximum possible score was 16 points). 14 days after the mice were injected with the anti-collagen antibody, the morphology of paws of the mice was recorded by photography, and the hind paws of the mice were scanned and recorded using a high-resolution micro-CT scanner.

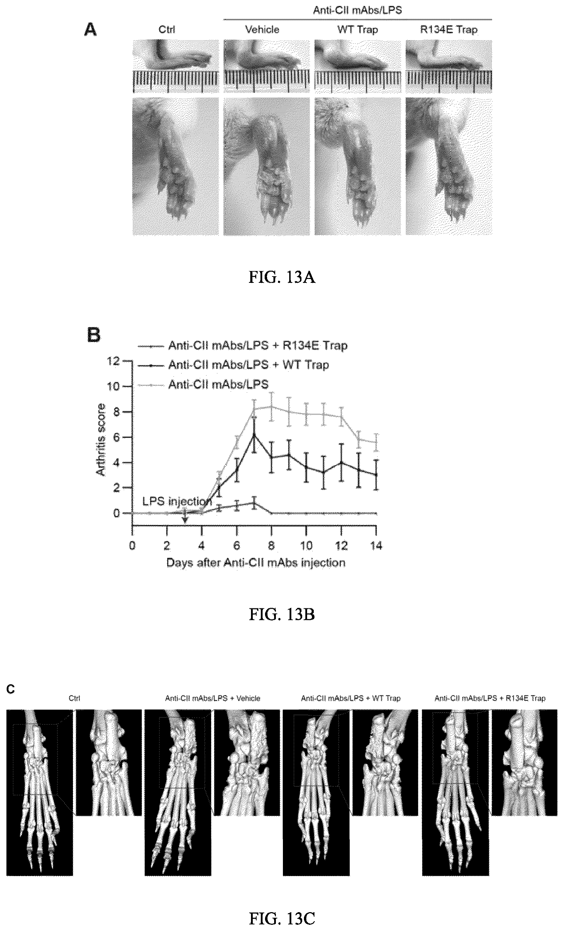

FIG. 13A shows representative photographs of the paws of mice in different drug treatment groups. The results indicate that stimulation with the anti-collagen antibody and LPS can significantly induce ankle and toe swelling in mice. The control wild-type IL-1 trap protein can partially alleviate ankle swelling, while the murine R134E mutant IL-1 trap protein can significantly reduce ankle swelling. FIG. 13B shows changes in the arthritis index of mice in different groups. The results indicate that after LPS stimulation, the arthritis index of mice increases rapidly. The three curves from top to bottom correspond to the control group without trap protein injection, the group injected with the wild-type IL-1 trap protein, and the group injected with the murine R134E mutant IL-1 trap protein, respectively. Injection of the control wild-type IL-1 trap protein can partially reduce the arthritis index in mice, while an equal dose of the murine R134E mutant IL-1 trap protein can almost completely inhibit the occurrence of arthritis in mice. FIG. 13C shows representative CT scan images of paws of mice in different groups. Stimulation with the anti-collagen antibody and LPS can significantly induce osteolytic lesions in the ankles of mice (with irregular bone spurs and deformities in the bone joints of the mice). The control wild-type IL-1 trap protein can partially alleviate the osteolytic lesions in the ankles of the mice, while the murine R134E mutant IL-1 trap protein can significantly inhibit the occurrence of osteolytic lesions in the ankles of the mice.

As described in Example 1 and Example 2, the present disclosure discloses a human IL-1R1 gene, a mutation carried in the gene is obtained by screening whole-exome sequencing data of a patient with an autoinflammatory disease, and a nucleotide mutation at human genome chromosome 2 locus chr2:102165209 (c.391A>G), is identified in the patient with the autoinflammatory disease, resulting in the K131E mutation in the translated human IL-1R1 protein. That is, there is a mutation at a single nucleotide site based on the wild-type IL-1R1 gene, i.e., nucleotides encoding the K131 site are mutated from AAA to GAA, resulting in a K131E mutation in the translated and expressed IL-1R1 protein. The gene sequence is as shown in SEQ ID No. 1; and this gene mutation is identified in a patient with an autoinflammatory disease, and its genetic diagnosis conforms to a dominant inheritance pattern. Subsequent mechanistic studies confirm that the K131E mutation weakens the interaction between IL-1R1 and its antagonistic molecule IL-1Ra, but does not affect the binding of IL-1R1 to its activating molecule IL-1β (as described in Example 4). This results in the inability to effectively inhibit IL-1R1-mediated inflammatory signals after reactivation. Therefore, this mutation is identified as a newly discovered rare pathogenic mutation for autoinflammatory diseases. The present disclosure discloses a human IL-1R1 protein, the human IL-1R1 protein is encoded by the human IL-1R1 gene and has a K131E mutation, i.e., the Lys131 site is mutated to Glu, and the amino acid sequence of the human IL-1R1 protein is as shown in SEQ ID No. 3.

As described in Example 3, the K131E mutation of the human IL-1R1 protein results in that the surface charge at the K131 site changes from positive to negative, and mutual attraction between the charge at the wild-type K131 site and the charge at the adjacent site on the antagonist IL-1Ra that interacts with the wild-type K131 site is changed to mutual repulsion. In addition, the K131D mutation also causes similar results, as described in Example 5 and Example 6. The present disclosure discloses a human IL-1R1 gene, where the nucleotide at the K131 site of the human IL-1R1 gene is mutated from AAA to GAC, resulting in a K131D mutation in the translated and expressed IL-1R1 protein, and the gene sequence is as shown in SEQ ID No. 2. The present disclosure discloses a human IL-1R1 protein, where the human IL-1R1 protein is artificially mutated to K131D based on the K131E mutation, i.e., an Lys131 site is mutated to Asp, and an amino acid sequence of the human IL-1R1 protein is as shown in SEQ ID No. 4.

The present disclosure discloses a diagnostic kit for an autoinflammatory disease, and the diagnostic kit is used for detecting a mutation in the human IL-1R1 gene, the mutation causes a mutation at the translated K131 site, the surface charge at the K131 site changes from positive to negative, and mutual attraction between the charge at the wild-type K131 site and the charge at the adjacent site on the antagonist IL-1Ra that interacts with the wild-type K131 site is changed to mutual repulsion after the mutation, and the K131 mutation includes a K131E or K131D mutation. The gene mutations include all possible changes in codons that code the amino acid at this site, which result in the K131E or K131D mutation, for example, the mutation from AAA to GAA, and the mutation from AAA to GAC. The diagnostic kit includes a nucleic acid amplification kit, a sequencing kit, or an immunoassay kit.

Use of the human IL-1R1 gene in preparation of a medicament is provided, the human IL-1R1 gene is used for preparation of a targeted gene modification therapeutic drug, including an RNA delivery drug, a CAR-T cell gene modification therapeutic drug, and a CRISPR-CAS9 gene modification drug; and the drug is used for treatment of related autoinflammatory diseases caused by the K131E mutation or K131D mutation of the IL-1R1 gene.

The human IL-1R1 protein carrying the K131E or K131D mutation disclosed according to the present disclosure has an amino acid sequence as shown in SEQ ID No. 3 or SEQ ID No. 4, and has an ability to bind to IL-1β and/or IL-1α without binding to IL-1Ra, as described in Example 4 and Example 6. If the protein is modified (by using, for example, a protein truncation or key amino acids involved in downstream signal transmission of mutated IL-1R1) to retain the protein's characteristics of binding to IL-1β and/or IL-1α without binding to IL-1Ra, while blocking an inflammatory signal transmission function of the protein, the protein can be used to inhibit the activity of IL-1β and/or IL-1α, to exert an anti-inflammatory effect *in vivo.* The protein sequence can be used for preparation of a protein drug or *in vitro* synthesis of a polypeptide inhibitor; and the drugs are used for treatment or prevention of diseases related to overexpression and activation of IL-1β and/or IL-1α, and specific blocking of functions of inflammatory activators IL-1β and/or IL-1α while retaining a function of the inflammatory antagonist IL-1Ra, and the indications of the drugs include but are not limited to: arthritis, gout, rheumatoid arthritis, Cryopyrin-Associated Periodic Syndromes (CAPS), scleroderma, diabetes, Familial Mediterranean Fever (FMF), Systemic Juvenile Idiopathic Arthritis (SJIA), Periodic Fever, Aphthous Stomatitis, Pharyngitis, and Adenitis Syndrome (PFAPA), Schnitzler syndrome, ankylosing spondylitis, recurrent multifocal osteomyelitis, cancer, dry eye, arteriosclerosis, and uveitis.

The present disclosure discloses a homodimeric protein specifically binding to IL-1α and/or IL-1β, and has a polypeptide sequence containing an amino acid sequence of SEQ ID No. 9 or SEQ ID No. 10, coded by a nucleotide sequence of SEQ ID No. 11 or SEQ ID No. 12. The homodimeric protein includes two homologous polypeptide sequences, where each polypeptide sequence includes three segments of amino acid sequences. The first segment of amino acid sequence includes amino acids at positions 21 to 359 of human IL-1RAP; the second segment of amino acid sequence includes amino acids at positions 18 to 332 of human IL-1R1, where the amino acid at position 131 is mutated from Lys to Glu or Asp, i.e., a K131E or K131D mutation; and the third segment of amino acid sequence includes a sequence of human immunoglobulin gamma-1 Fc. A structure of the homodimeric protein is the same as that described in Example 7. As described in Example 7, the homodimeric protein with K131E mutation has a binding activity to human IL-1Ra with an EC50 of 344 ng/mL as determined by enzyme-linked immunosorbent assay. However, the mutation does not affect the binding ability of the homodimeric protein to IL-1α and/or IL-1β. As described in Example 8, the affinity constant of the homodimeric protein with the K131E mutation to IL-1Ra is 5.069 × 10⁻⁹ M, which is significantly lower than that of the homodimeric protein without the mutation. This demonstrates that this mutation is a functional mutation that inhibits the binding activity of the homodimeric trap protein to IL-1Ra. The verification of the function is consistent with that described in Example 9. In the presence of IL-1Ra, the IL-1 trap protein with a K131E mutation shows a stronger ability to inhibit *in vitro* the activation of intracellular NF-κB signaling pathway in human embryonic kidney cell lines in response to IL-1β than the unmutated trap protein. As described in Example 10, the homodimeric protein with K131E mutation inhibits *in vitro* the production of human IL-6 in human lung fibroblasts in response to the treatment with human IL-1β, with an IC50 value of 118.2 pM. The inhibitory capacity is higher than that of the homodimeric protein without the mutation and the common IL-1β inhibitos, which proves that this mutation plays an important role in enhancing the function of the homodimeric protein.

Therefore, the homodimeric protein specifically binding to IL-1α and/or IL-1β disclosed according to the present disclosure can be directly used for synthesis of polypeptides *in vitro* and used for preparation of drugs, where the drugs are used for treatment or prevention of diseases related to overexpression and activation of IL-1α and/or IL-1β, and specific blocking of the functions of inflammatory activators IL-1α and/or IL-1β while retaining the function of the inflammatory antagonist IL-1Ra, and the indications of the drugs include but are not limited to: arthritis, gout, rheumatoid arthritis, Cryopyrin-Associated Periodic Syndromes (CAPS), scleroderma, diabetes, Familial Mediterranean Fever (FMF), Systemic Juvenile Idiopathic Arthritis (SJIA), Periodic Fever, Aphthous Stomatitis, Pharyngitis, and Adenitis Syndrome (PFAPA), Schnitzler syndrome, ankylosing spondylitis, recurrent multifocal osteomyelitis, cancer, dry eye, arteriosclerosis, and uveitis.

As described in Examples 11 and 12, the mice were used as an animal model, the murine homodimeric protein carrying the R134E mutation, which was conserved with the foregoing homodimeric protein with the K131E mutation, was constructed. This homodimeric protein has a polypeptide sequence containing the amino acid sequence of SEQ ID No. 17 or SEQ ID No. 18 encoded by the nucleic acid sequence of SEQ ID No. 19 or SEQ ID No. 20, thereby exerting a significant inhibitory effect on the production of mIL-6 induced by mIL-1β and LPS in mice. In addition, the inhibitory effect of the homodimeric protein carrying the R134 mutation was more obvious, demonstrating that the function of this site was similar to that of the human K131 site. As described in Example 13, in the mouse arthritis model, the homodimeric protein with the R134E mutation showed a more significant alleviation effect on the collagen antibody-induced arthritis model, which demonstrated the specific function of this mutation and the potential of this homodimeric protein in treating arthritis-related diseases.

The technical features in the foregoing examples can be further combined. For brevity of description, not all possible combinations of various technical features in the foregoing examples are described. However, as long as there is no contradiction between the combinations of these technical features, the combinations should be considered as falling within the recorded scope of this description.

Only several embodiments of the present disclosure are described in the foregoing examples. The descriptions are relatively detailed and specific, but cannot be construed as a limitation on the scope of the present disclosure. It should be noted that a person of ordinary skills in the art could also make several variations and improvements without departing from the concept of the present disclosure. These variations and improvements all fall within the protection scope of the present disclosure. Therefore, the protection scope of the present disclosure shall be limited by the appended claims.

## Claims

1. A human IL-1R1 gene, wherein the human IL-1R1 gene comprises a mutation at a single nucleotide site based on a wild type, in which nucleotides encoding a K131 site are mutated from AAA to GAA, resulting in a K131E mutation in a translated and expressed IL-1R1 protein, and has a gene sequence as shown in SEQ ID No. 1; or wherein the human IL-1R1 gene comprises a mutation at a single nucleotide site based on a wild type, in which nucleotides encoding a K131 site mutated from AAA to GAC, resulting in a K131D mutation in a translated and expressed IL-1R1 protein, and has a gene sequence as shown in SEQ ID No. 2.

2. The human IL-1R1 gene according to claim 1, wherein the K131E mutation is obtained by screening whole-exome sequencing data of a patient with an autoinflammatory disease, wherein a nucleotide mutation at human genome chromosome 2 locus chr2:102165209, c.391A>G, is identified in the patient, which causes the K131E mutation in the translated human IL-1R1 protein.

3. A human IL-1R1 protein, wherein the human IL-1R1 protein is encoded by the human IL-1R1 gene according to claim 1, has a K131E mutation in which a Lys131 site is mutated to Glu, and has an amino acid sequence as shown in SEQ ID NO. 3; or the human IL-1R1 protein has an artificial mutation based on the K131E mutation, referred to as a K131D mutation, in which a Lys131 site is mutated to Asp, and has an amino acid sequence as shown in SEQ ID NO. 4.

4. The human IL-1R1 protein according to claim 3, wherein the human IL-1R1 protein is obtained by protein three-dimensional structure simulation technology, wherein a wild-type K131 site is located at a interaction interface between the IL-1R1 protein and its antagonist IL-1Ra; after the K131 site is mutated to E or D, a surface charge at the K131 site changes from positive to negative, and mutual attraction between a charge at the wild-type K131 site and a charge at an adjacent site on the antagonist IL-1Ra that interacts with the wild-type K131 site is changed to mutual repulsion after the mutation.

5. The human IL-1R1 protein according to claim 3, wherein in an *in vitro* co-immunoprecipitation assay, the K131E mutation causes weakened interaction between the human IL-1R1 protein and IL-1Ra, but does not affect binding of the IL-1R1 protein to IL-1β and/or IL-1α.

6. Use of the human IL-1R1 gene according to claim 1 in preparation of a medicament, wherein the human IL-1R1 gene is used for preparation of a targeted gene modification therapeutic drug, including an RNA delivery drug, a CAR-T cell gene modification therapeutic drug, or a CRISPR-CAS9 gene modification drug; and the medicament is used for treatment of related autoinflammatory diseases clinically diagnosed as caused by the K131E mutation or K131D mutation of the IL-1R1 gene.

7. Use of the human IL-1R1 protein according to claim 3 or a polypeptide fragment thereof in preparation of a medicament, wherein the human IL-1R1 protein or the polypeptide fragment thereof is used for preparation of a protein drug or *in vitro* synthesis of a polypeptide inhibitor, wherein the medicament is used for treatment or prevention of diseases related to overexpression and activation of IL-1α and/or IL-1β, specifically blocking functions of inflammatory activators IL-1α and/or IL-1β while retaining a function of an inflammatory antagonist IL-1Ra, and indications of the medicament include but are not limited to: arthritis, gout, rheumatoid arthritis, Cryopyrin-Associated Periodic Syndromes (CAPS), scleroderma, diabetes, Familial Mediterranean Fever (FMF), Systemic Juvenile Idiopathic Arthritis (SJIA), Periodic Fever, Aphthous Stomatitis, Pharyngitis, and Adenitis Syndrome (PFAPA), Schnitzler syndrome, ankylosing spondylitis, recurrent multifocal osteomyelitis, cancer, dry eye, arteriosclerosis, and uveitis.

8. A diagnostic kit, wherein the diagnostic kit is used for detecting a mutation in a human IL-1R1 gene or a human IL-1R1 protein, wherein the mutation causes a mutation at a translated K131 site, so that a surface charge at the K131 site changes from positive to negative, and mutual attraction between a charge at the wild-type K131 site and a charge at an adjacent site on an antagonist IL-1Ra that interacts with the wild-type K131 site is changed to mutual repulsion after the mutation, wherein the K131 mutation includes a K131E or K131D mutation, and wherein the diagnostic kit includes a nucleic acid amplification kit, a sequencing kit, or an immunoassay kit.

9. A homodimeric protein specifically binding to IL-1α and/or IL-1β, wherein the homodimeric protein is also referred to as an IL-1 trap protein, which comprises two homologous polypeptide sequences, each polypeptide sequence comprising three segments of amino acid sequences:
a first segment of amino acid sequence including amino acids at positions 21 to 359 of human IL-1RAP, and the first segment of amino acid sequence is as shown in SEQ ID No. 5;
a second segment of amino acid sequence including amino acids at positions 18 to 332 of human IL-1R1, wherein an amino acid at position 131 is mutated from Lys to Glu or Asp, referred to as a K131E or K131D mutation, and the second segment of amino acid sequence is as shown in SEQ ID No. 6 or SEQ ID No. 7; and
a third segment of amino acid sequence including a sequence of human immunoglobulin gamma-1Fc, and the third segment of amino acid sequence is as shown in SEQ ID No. 8.

10. The homodimeric protein specifically binding to IL-1α and/or IL-1β according to claim 9, wherein the homodimeric protein having the K131E mutation has a binding activity to human IL-1Ra with an EC50 of 344 ng/mL as determined by enzyme-linked immunosorbent assay.

11. The homodimeric protein specifically binding to IL-1α and/or IL-1β according to claim 9, wherein the homodimeric protein having the K131E mutation has an affinity constant of 5.069×10⁻⁹ M for IL-1Ra.

12. The homodimeric protein specifically binding to IL-1α and/or IL-1β according to claim 9, wherein in the presence of IL-1Ra, the IL-1 trap protein having the K131E mutation has a stronger ability to inhibit activation of intracellular NF-κB signaling pathway in human embryonic kidney cell lines in response to IL-1β *in vitro* as compared to an unmutated trap protein.

13. The homodimeric protein specifically binding to IL-1α and/or IL-1β according to claim 9, wherein the homodimeric protein having the K131E mutation inhibits production of human IL-6 in human lung fibroblasts *in vitro* in response to treatment with human IL-1β, with an IC50 value of 118.2 pM.

14. The homodimeric protein specifically binding to IL-1α and/or IL-1 β according to claim 9, wherein the polypeptide sequence comprises an amino acid sequence of SEQ ID No. 9 or SEQ ID No. 10.

15. An isolated nucleic acid, wherein the isolated nucleic acid encodes a polypeptide comprising an amino acid sequence of SEQ ID No. 9 or SEQ ID No. 10.

16. The nucleic acid according to claim 15, wherein a nucleic acid sequence of the nucleic acid comprises SEQ ID No. 11 or SEQ ID No. 12.

17. Use of the homodimeric protein specifically binding to IL-1α and/or IL-1β according to claim 9 in preparation of a medicament, wherein the medicament is used for treatment or prevention of diseases related to overexpression and activation of IL-1α and/or IL-1β, specifically blocking functions of inflammatory activators IL-1α and/or IL-1β while retaining a function of an inflammatory antagonist IL-1Ra, and indications of the medicament include but are not limited to: arthritis, gout, rheumatoid arthritis, Cryopyrin-Associated Periodic Syndromes (CAPS), scleroderma, diabetes, Familial Mediterranean Fever (FMF), Systemic Juvenile Idiopathic Arthritis (SJIA), Periodic Fever, Aphthous Stomatitis, Pharyngitis, and Adenitis Syndrome (PFAPA), Schnitzler syndrome, ankylosing spondylitis, recurrent multifocal osteomyelitis, cancer, dry eye, arteriosclerosis, and uveitis.

18. A homodimeric protein capable of specifically binding to mouse IL-1α and/or mouse IL-1β, wherein the homodimeric protein is also referred to as a mouse IL-1 trap protein, which includes two homologous polypeptide sequences, each polypeptide sequence comprising three segments of amino acid sequences:
a first segment of amino acid sequence including amino acids at positions 21 to 359 of mouse IL-1RAP, and the first segment of amino acid sequence is as shown in SEQ ID No. 13;
a second segment of amino acid sequence including amino acids at positions 24 to 336 of mouse IL-1R1, wherein the amino acid at position 134 is mutated from Arg to Glu or Asp, referred to as an R134E or R134D mutation, and the second segment of amino acid sequence is as shown in SEQ ID No. 14 or SEQ ID No. 15; and
a third segment of amino acid sequence including a sequence of mouse immunoglobulin gamma-2A Fc, and the third segment of amino acid sequence is as shown in SEQ ID No. 16.

19. The homodimeric protein according to claim 18, wherein the homodimeric protein inhibits IL-1 β-induced mouse IL-6 production in mice, and under the same dosage, the mouse IL-1 trap protein carrying the R134E mutation has a superior inhibitory effect as compared to an unmutated mouse IL-1 trap protein.

20. The homodimeric protein according to claim 18, wherein the homodimeric protein inhibits LPS-induced mouse IL-6 production in mice, and under the same dosage, the mouse IL-1 trap protein carrying the R134E mutation has a superior inhibitory effect as compared to an unmutated mouse IL-1 trap protein.

21. The homodimeric protein according to claim 18, wherein the homodimeric protein alleviates an arthritis phenotype in collagen antibody-induced rheumatoid arthritis model mice, and under the same dosage, the mouse IL-1 trap protein carrying the R134E mutation has a superior inhibitory effect as compared to an unmutated mouse IL-1 trap protein.

22. The homodimeric protein according to claim 18, wherein the polypeptide sequence comprises an amino acid sequence of SEQ ID No. 17 or SEQ ID No. 18.

23. An isolated nucleic acid, wherein the nucleic acid encodes a polypeptide comprising an amino acid sequence of SEQ ID No. 17 or SEQ ID No. 18.

24. The nucleic acid according to claim 23, wherein a nucleic acid sequence of the nucleic acid comprises SEQ ID No. 19 or SEQ ID No. 20.
